# EUROPÄISCHE PATENTSCHRIFT

(11) **EP 2 575 844 B1**
(45) Veröffentlichungstag und Bekanntmachung des Hinweises auf die Patenterteilung: **23.11.2016**
(21) Anmeldenummer: 11723430.2
(22) Anmeldetag: 30.05.2011
(51) Int. Cl.: A61K 36/87, A61P 9/00

(54) **VERFAHREN ZUR HERSTELLUNG EINES ANGEREICHERTEN EXTRAKTES AUS BLÄTTERN VON WEINREBENGEWÄCHSEN**
Method for producing an enriched extract from vine plants
Procédé de fabrication d'un extrait enrichi constitué de feuilles de vignes

(30) Priorität: 28.05.2010 EP 10164253
(43) Veröffentlichungstag der Anmeldung: 10.04.2013
(73) Patentinhaber: Finzelberg GmbH & Co. KG, 56626 Andernach (DE)
(72) Erfinder: FEISTEL, Björn, 56626 Andernach (DE); WALBROEL, Bernd, 53639 Königswinter (DE)
(74) Vertreter: dompatent von Kreisler Selting Werner - Partnerschaft von Patent- und Rechtsanwälten mbB
(86) Internationale Anmeldenummer: PCT/EP2011/058808
(87) Internationale Veröffentlichungsnummer: WO 2011/147989

(56) Entgegenhaltungen:
- WO-A1-01/28363
- WO-A1-2009/135880
- DATABASE WPI Week 200144 Thomson Scientific, London, GB; AN 2001-409576 XP002592483, & CN 1 288 892 A (SONG X) 28. März 2001 (2001-03-28)
- KIESEWETTTER H ET AL: "EFFICACY OF ORALLY ADMINISTERED EXTRACT OF RED VINE LEAF AS 195 (FOLIA VITIS VINIFERAE) IN CHRONIC VENOUS INSUFFICIENCY (STAGES I-II) A RANDOMIZED, DOUBLE-BLIND, PLACEBO-CONTROLLED TRIAL", ARZNEIMITTEL FORSCHUNG. DRUG RESEARCH, ECV EDITIO CANTOR VERLAG, AULENDORF, DE, Bd. 50, Nr. 2, 1. Januar 2000 (2000-01-01) , Seiten 109-117, XP001146699, ISSN: 0004-4172
- META MAHENDRADATTA, GEORG SCHWEDT: "Sample preparation for photometric determination of histamine in foodstuffs compared with capillary electrophoresis", ZEITSCHRIFT FUER LEBENSMITTEL-UNTERSUCHUNG UND -FORSCHUNG A, Bd. 20, Nr. 4, 1998, Seiten 246-250, XP009136366, ISSN: 1431-4630
- KREIS W: "Secondary plant products and cancer / Sekündare Planzenstoffe und Krebs", DEUTSCHE ZEITSCHRIFT FUER ONKOLOGIE, KARL F. HAUG VERLAG IN MVS MEDIZINVERLAGE STUTTGART GMBH & CO. KG, DE, vol. 41, no. 3, 1 January 2009 (2009-01-01), pages 100-108, XP009182796, ISSN: 1617-5891, DOI: 10.1055/S-0029-1213576

## Beschreibung

Die vorliegende Erfindung betrifft Extrakte aus Blättern von Weinrebengewächsen, ihre Herstellung und Verwendung.

Unter der Bezeichnung Venenschwäche wird umgangssprachlich ein Krankheitsbild verstanden, bei dem es zu Wassereinlagerungen in den unteren Gliedmaßen kommt und sich durch Befindlichkeitsstörungen wie schweren und schnell ermüdenden Beinen äußert. Das sogenannte Versacken von Wasser in den Beinen bezeichnet eine ödematöse Ansammlung von Wasser im Gewebe und setzt voraus, dass Wasser aus den Blutgefäßen übertreten kann. Es ist demnach anzunehmen, dass die Permeabilität der Gefäße übermäßig gesteigert ist.

Die Venenschwäche kann als ein frühes Stadium der Chronischen Venösen Insuffizienz (CVI) gesehen werden. Die CVI, auch chronisch-venöses Stauungssyndrom oder Chronische Veneninsuffizienz genannt, beruht auf einer Mikrozirkulationsstörung der Gefäße infolge einer venösen Abflussbehinderung. Das Risiko, an einer chronisch-venösen Insuffizienz zu leiden, steigt mit Alter, Fettleibigkeit, einer Anamnese mit Venenentzündungen, tiefer Venenthrombose und schwerem Beintrauma. CVI kommt bei Frauen doppelt so häufig vor wie bei Männern.

Wichtig für die Entstehung einer chronisch-venösen Insuffizienz ist das Vorhandensein eines pathologischen Bluthochdrucks der oberflächlichen Venen. Normal ist dieser bei 20-30 mmHg, steigt jedoch durch venöse Thrombose, primär oder sekundär pathologische Venenklappen oder abgeschwächte Muskelpumpe auf 60-90 mmHg. Dieser Hochdruck verursacht die für die späteren Komplikationen verantwortlichen anatomischen, physiologischen und histologischen Veränderungen der Gefäße. Unter physiologischen Bedingungen wird das Kapillarbett vor starken Druckveränderungen geschützt, da präkapilläre Arteriolen sich reflektorisch kontrahieren können. Diesen Reflex scheinen Patienten mit chronisch erhöhtem venösen Druck jedoch verloren zu haben, d.h. dass bei Veränderungen der Körperlage Druckveränderungen direkt an das Kapillarbett weiter gegeben werden.

Bei dauerhaft erhöhtem venösen Druck entstehen Veränderungen, die die Kapillarwände betreffen. Dazu gehören eine Verbreiterung und Verlängerung des Kapillarbetts, erhöhte Endotheloberfläche, erhöhte Einlagerung von Kollagen IV in die Basalmembran und perikapilläre Einlagerung von Fibrin. Die Zusammenwirkung von erhöhtem venösen Druck und abnormen Kapillaren mit erhöhter Permeabilität führen zu einem Ausschwemmen von Wasser, großen Proteinen und roten Blutzellen in das Interstitium. Das sich ansammelnde Fibrin scheint auch weniger einfach gelöst werden zu können. Gleichzeitig kommt es zu einem verminderten Gasaustausch, also einer kutanen Hypoxie. Dies führt unter anderem zu einer Leukozytenaktivierung.

Einen ähnlichen Prozess kennt man aus der Akut-Phase-Reaktion von Entzündungen. Hierbei wird die Permeabilität der Gefäßwände durch Gefäßmediatoren, wie Histamin, Prostaglandine (z.B. PGE-2), Kinine und Serotonin, für wenige Minuten gesteigert. Typische Auslöser einer solchen Akut-PhasenReaktion sind Zytokine wie Interleukin-1 (IL-1ß), Interleukin-6 (IL-6), Tumornekrosefaktor Alpha (TNF-α), Tumorwachstumsfaktor Beta (TGF-ß) oder Interferon-Gamma die unter anderem von Endothelzellen, Fibroblasten, Makrophagen, Granulozyten und Lymphozyten freigesetzt werden. Diese immunkompetenten Zellen setzen die Zytokine beispielsweise nach der Beschädigung oder dem Absterben benachbarter Zellen frei. Die Zytokine gelangen über die Blutbahn in die Leber wo sie zusammen mit Cortisol das Organ zur Produktion von etwa 30 verschiedenen Akut-Phasen-Proteinen (APP) anregt. Die APPs begünstigen die Wundheilungsprozesse und wirken der Gewebeschädigung entgegen. Die Auswirkungen der Akut-Phase-Reaktion sind sowohl lokal, als auch systemisch. Die lokalen Effekte sollen mögliche Infektionen begrenzen und Xenobiotika (zum Beispiel synthetisch hergestellte Farbstoffe, Pestizide und chlorierte Lösungsmittel) eliminieren. Tote oder geschädigte Zellen werden per Phagozytose beseitigt und Reparaturprozesse initiiert. Dagegen sind die systemischen Auswirkungen Abwehrreaktionen, die das Überleben des Gesamtorganismus sicherstellen sollen, beispielsweise gegen Endotoxine.

Entsprechend der Leitlinien der Deutschen Gesellschaft für Phlebologie (AWMF-Leitlinien-Register Nr. 037/009) werden unter anderem als adjuvante Pharmakotherapeutika in der Behandlung der Chronischen Venösen Insuffizienz folgende Mittel eingesetzt: Acetylsalicylsäure, Diuretika, Fibrinolytika (Defibrotide, Stanazolol, Sulodexide), Fibrinolyse-Verstärker, Iloprost, Pentoxyphyllin, Prostaglandin E1, Saponine (Extrakte aus Centella, Hippocastanus, Ruscus), Benzaron, Calcium-Dobesilat, Naftazone, Tribenoside, Ginkgo biloba sowie zahlreiche Substanzen, die der großen Gruppe der Flavonoide angehören [Havsteen BH. the biochemistry and medical significance of the flavonoids. Pharmacol Ther 2002;96(2-3):67-202]. Ein eindeutig positiver Effekt der Flavonoide auf die Ödementwicklung konnte nachgewiesen werden [Martinez MJ, Bonfill X, Moreno RM, Vargas E, Capellà D. Phlebotonics for venous insufficiency. Cochrane Database of Systematic Reviews 2005, Issue 3. Art. No.: CD003229. DOI: 10.1002/14651858.CD003229.pub2.].

Es hat sich gezeigt, dass in Weinbauregionen das Krankheitsbild der Venenschwäche deutlich geringer ausfällt. Dies legt nahe, dass in der Pflanze *Vitis vinifera* L. Verbindungen enthalten sein müssen, welche antagonistisch der Erkrankung entgegenwirken. Traditionell wurden Weinblätter sowohl als Gemüse gegessen, als auch in Form von Dekokten und Infusen medizinisch angewendet bei menopausalen Störungen oder zur Behandlung von Hämorriden. In der französischen Pharmakopoe ist ein Extrakt aus Rotweinlaub als Venentonikum empfohlen. Seit einigen Jahren sind unter dem Namen Antistax® Präparate mit wässrigem Extrakt aus rotem Weinlaub im Markt, die erfolgreich bei Chronisch-venöser Insuffizienz (CVI) eingesetzt werden [Kiesewetter H, Koscielny J, Kalus U, Vix JM, Peil H, Petrini O, et al. Efficacy of orally administered extract of red vine leaf AS 195 (folia vitis viniferae) in chronic venous insufficiency (stages I-II). A randomized, double-blind, placebo-controlled trial. Arzneimittel-Forschung 2000;50(2):109-117]. Der hierin enthaltene Extrakt ist durch einen Gehalt von etwa 5% Flavonoiden gekennzeichnet. Als eine weitere Gruppe phenolischer Verbindungen sind etwa 0,1% Anthocyane enthalten.

Entsprechend der guten Verträglichkeit der bisher bekannten Weinlaubextrakte, war es Ziel der vorliegenden Erfindung einen neuartigen Extrakt bereitzustellen, der sich ebenso eignet für eine Verwendung im gesamten Bereich entzündlicher Erkrankungen. Lokale anti-inflammatorische Effekte können gegebenenfalls entlang des gesamten gastrointestinalen Traktes hilfreich bei der Behandlung oder Prophylaxe entzündlicher Erkrankungen sein. Gingivitis, Parondontitis, Stomatitis, Ösophagitis und Gastritis beschreiben die entzündlichen Erkrankungen des oberen Teils des Verdauungstraktes. Lymphozytäre Colitis, Colitis ulcerosa und Morbus Crohn sind die bekanntesten Vertreter entzündlicher Erkrankungen des Intestinalbereiches.

Unter Hämorrhoiden versteht man umgangssprachlich die Krampfaderähnliche oder auch knotenförmige Schwellung bzw. Erweiterung eines arteriovenösen Gefäßpolsters, das zwischen dem Enddarm und dem Schließmuskel des Afters liegt. Dieses Gefäßpolster wird auch als Plexus hämorrhoidalis bezeichnet. Es besteht aus einem Arterien- und Venengeflecht, das gemeinsam mit dem Schließmuskel den Feinverschluss des Afters ermöglicht. Wenn von Hämorrhoiden gesprochen wird, sind damit aber meist Hämorrhoidalleiden gemeint. Typische Symptome sind leichte Blutungen, Druckgefühl, Juckreiz, Hautausschlag und im fortgeschrittenen Stadium Probleme beim kontrollierten Abgang von Darmgasen und Stuhl. Ursachen können u.a. Krampfadern-ähnliche Aussackungen anusnaher Blutgefäße sein. Eine ähnliche Erkrankung mit der Bezeichnung Perianalthrombose (= unechte Hämorrhoiden) entsteht durch Ausbildung eines Blutgerinnsels (Thrombus) in den Venen der Analhaut und wird auch Analthrombose genannt. Perianalthrombosen entstehen oft durch langes Sitzen, dem Sitzen auf kalten Flächen, durch starkes oder langes Pressen beim Stuhlgang (z. B. bei hartem Stuhl) sowie während der Schwangerschaft und der Geburt. Sie können auch durch starken Husten, körperliche Belastung, Flüssigkeitsmangel durch zu wenig trinken und sogar durch Niesen hervorgerufen werden. Auch hierbei können Blutgefäße über das übliche Maß hinaus gedehnt werden. Sowohl bei Hämorrhoidalleiden, als auch bei Perianalthrombose ist neben operativen Maßnahmen die Gabe von Suppositorien mit schmerzstillenden oder antientzündlichen Wirkstoffen gängig (Leitlinie zu Hämorroidalleiden "www.uni-duesseldorf.de/AWMF/II/081-007p.htm" und Leitlinie zu Analthrombose "www.derma.de/585.0.html").

Für andere Pflanzenteile von Vitis vinifera L., beispielsweise den Traubenkernen, sind bereits einige Anreicherungsmethoden beschrieben. Während Verfahren wie die Mikrowellenextraktion (CN1102589C) aber nur für geringe Anreicherungsfaktoren durch Mehrausbeute sorgen, sind Extraktionen mit überkritischen Fluiden (CN1107110C) nur für lipophile Strukturen geeignet. CN1454896 verwendet eine saure Primärextraktion in Verbindung mit einer Flüssig-Flüssig-Extraktion zur Anreicherung oligomerer Proanthocyanidine. EP348781 beschreibt ein Anreicherungsverfahren für oligomere phenolische Verbindungen aus Traubenkernen mittels einer Membranfiltration, während WO 2005036988 und EP1909750 ein chromatographisches Verfahren zur selektiven Anreicherung von Polyphenolen und Abreicherung von Monomeren beanspruchen.

GB 934554 beschreibt eine warme alkoholisch-wässrige Extraktion (50-80% EtOH) von Rotweinlaub, sowie eine anschließende Ansäuerung zum Erhalt der roten Farbe. Zur Entfernung von Chlorophyll und Wachsen wird eine Flüssig-Flüssig-Extraktion mittels Benzol oder Tetrachlorkohlenstoff vorgeschlagen, alternativ eine Umfällung in reinem Wasser. Durch Aussalzen kann die Präzipitation der Gerbstoffe beschleunigt werden. Es folgt ein Aufreinigungsschritt über eine Flüssig-Flüssig-Extraktion mit Butanol oder Pentanol. Aus der resultierenden alkoholischen Extraktphase wird optional mit Ether, Petroleum oder Benzol eine Procyanidin-Fraktion ausgefällt und anschließend schonend getrocknet (im Vakuum oder auf einem Sprühturm). Dieses Verfahren ist sehr aufwendig und kostenintensiv und das resultierende Produkt hat sehr starke Abweichungen vom Referenzextrakt. Daher wird ein alternativer Herstellprozess gesucht, der eine kostengünstigere Herstellung erlaubt.

Aufgabe der vorliegenden Erfindung war es, alternative wirksame Extrakte von Weinrebengewächsen bereit zu stellen.

Gelöst wird die Aufgabe durch ein Verfahren zur Herstellung eines Extraktes aus Blättern von Pflanzen der Ordnung *Vitales* umfassend folgende Schritte:
a) Bereitstellen einer Droge aus Blättern von Pflanzen der Ordnung *Vitales*
b) Extraktion der Droge mit einem Eluenten ausgewählt aus Wasser und Gemischen von Wasser mit Alkohol, um einen Rohextrakt zu erhalten,
c) Abtrennen des Rohextraktes von der extrahierten Droge,
d) zumindest teilweises Entfernen des Eluenten, um einen Dickextrakt zu erhalten,
e) Rücklösen des Dickextraktes in Wasser,
f) Abtrennen von unlöslichen Bestandteile,
g) selektive Anreicherung sekundärer Pflanzeninhaltsstoffe durch
   i. eine 2-Phasenextraktion als Festphasenextraktion mit einem Adsorberharz ausgewählt aus der Gruppe der nichtionischen hydrophoben Divinylbenzen-Copolymere, aliphatischen Esterpolymere und Formophenolpolymere mit einer durchschnittlichen inneren Porengröße von 30 bis 70 nm als feste Phase, wobei die Substanzen, die an den Adsorber binden eluiert werden und weiter verwendet werden
      oder
   ii. eine 2-Phasenextraktion als Flüssig-Flüssig-Extraktion wobei die zweite Phase eine mit Wasser nicht mischbare Flüssigkeit ist, ausgewählt aus der Gruppe der Alkohole, Ketone, Alkane oder Ester aus und die Fraktion, die in die zweite Phase übertritt weiter verwendet wird
      so dass der Extrakt mindestens 15 Gew.-% an Flavonoiden und/oder mindestens 0,2 Gew.-% Anthocyane, bezogen auf den Trockenextrakt, enthält.

Erfindungsgemäß werden Blätter von Pflanzen der Ordnung Vitales eingesetzt. Diese können prinzipiell frisch oder getrocknet eingesetzt werden.

In einer Ausführungsform werden als Drogen Blätter der Pflanze Vitis Vinifera eingesetzt

Extrakt im Sinne dieser Anmeldung ist ein Drogenauszug, der mittels Extraktion gewonnen wird. Es handelt sich um Vielstoffgemische, die eine Vielzahl von Einzelsubstanzen enthalten (typischerweise mehr als 10) und ein Teilaspekt der Inhaltsstoffe abbilden. In einigen Fällen sind Leitsubstanzen bekannt, die zur Quantifizierung eines Extraktes verwendet werden können, ohne dass diese zwangsläufig wirksame Komponenten sein müssen. Bei einem Drogenauszug ergibt sich das Wirkprofil aus der Gesamtzusammenstellung der Inhaltsstoffe.

Es wird bevorzugt, dass die Blätter vor der Extraktion zerkleinert werden.

In einer speziellen Ausführungsform der vorliegenden Erfindung erfolgt die Bereitstellen der Droge durch Sammeln von Blättern von Pflanzen der Ordnung Vitales zu einem Zeitpunkt, an dem der Flavonoidgehalt ein Optimum erreicht, Trocknen und Zerkleinern der Blätter sowie optional durch Schneiden der Blätter in Stücke.

Die Extraktion erfolgt mit Wasser oder Gemischen von Wasser mit Alkohol, um einen Rohextrakt zu erhalten. Besonders geeignet sind Mengen von 5 bis 30 kg Extraktionsmittel pro 1 kg getrockneter Droge.

Bevorzugt wird als Eluent Wasser oder eine Ethanolwasser-Mischung eingesetzt, die 5 bis 95% (V/V) Ethanol enthält.

Noch mehr bevorzugt ist der Einsatz eines Eluenten, der neben Wasser, Ethanol in einer Menge von 25 bis 50 % (V/V) enthält.

Bevorzugt wird die Extraktion bei einer Temperatur im Bereich von 40 bis 80° durchgeführt, wobei sich ein Temperaturbereich von 15 bis 25°C unterhalb der Siedetemperatur des verwendeten Eluenten besonders bewährt hat.

Der bei der Extraktion vorherrschende Druck ist über weite Bereiche unkritisch. Üblicherweise wird man die Extraktion jedoch bei einem Druck im Bereich von 900 bis 1500 mbar, bevorzugt von 950 bis 1100 mbar und insbesondere bei Umgebungsdruck 1013 mbar durchführen. Der Rohextrakt wird von der extrahierten Droge abgetrennt.

Das Abtrennen erfolgt üblicherweise durch Filtration oder andere dem Fachmann bekannte Verfahren. Beispielsweise eignet sich eine Filtration über eine Sieblochplatte mit 4mm-Löchern und nachgeschalteten Polyamid-Siebbeuteln mit 250µm Maschenweite. Weitere geeignete Verfahren hierfür sind dem Fachmann bekannt.

Anschließend wird der Eluent aus dem abgetrennten Rohextrakt zumindest teilweise unter Erhalt eines Dickextraktes entfernt. Unter zumindest teilweiser Entfernung des Eluenten ist im Rahmen der vorliegenden Erfindung eine Entfernung des Eluenten von 30 bis 99 % (V/V) bezogen auf das eingesetzte Eluentenvolumen, bevorzugt 75 bis 98 % (V/V) und insbesondere eine im Wesentlichen vollständige Entfernung des Eluenten zu verstehen, d.h. eine Entfernung von wenigstens 95 % (V/V). Üblicherweise wird man den Eluenten durch Erwärmen und/oder unter vermindertem Druck entfernen. Geeignete Methoden und Vorrichtungen hierfür sind dem Fachmann bekannt.

Der so erhaltene Dickextrakt wird in Wasser rückgelöst und unlösliche Bestandteile werden abgetrennt.

Anschließend erfolgt eine selektive Anreicherung sekundärer Pflanzeninhaltsstoffe durch eine 2-Phasen-Extraktion Die 2-Phasen-Extraktion kann als Fest-Phasen-Extraktion durchgeführt werden, wobei als feste Phase ein Adsorberharz aus der Gruppe der nichtionischen hydrophoben Divinylbenzen-Copolymere, aliphatischen Esterpolymere und Formophenolpolymere verwendet wird. Solche sind unter den Bezeichnungen Amberlite^{®}, Levatite^{®}, Miontech^{®} und Diaion^{®} kommerziell verfügbar. Die für das erfindungsgemäße Verfahren geeigneten Adsorberharze sind durch eine durchschnittliche innere Porengröße im Bereich von 300 - 700 Angström, bevorzugt von 400 - 500 Angström, gekennzeichnet.

Die Substanzen, die an den Adsorber binden, werden eluiert und weiter verwendet; die nicht gebundenen Substanzen werden verworfen.

Üblicherweise wird man die Extraktion in einem Temperaturbereich von 10 bis 30°C durchführen. Die verwendete Aufgabelösung wird üblicherweise zwischen pH 3 und pH 6 eingestellt, bevorzugt zwischen pH 4,5 und pH 5,5, besonders bevorzug auf pH 5.

Es kann sich bei der 2-Phasen-Extraktion auch um eine Flüssig-Flüssig-Extraktion handeln, wobei die zweite Phase eine mit Wasser nicht mischbare Flüssigkeit, ausgewählt aus der Gruppe der Alkohole, Ketone, Alkane oder Ester ist. In einer speziellen Ausführungsform ist die zweite Phase ein mit Wasser nicht mischbarer Alkohol, bevorzugt ein C₄-C₅-Alkohol und besonders bevorzugt n-Butanol. Hierbei wird die Fraktion, die in die 2. Phase übertritt weiterverwendet und die wässrige Phase verworfen.

Üblicherweise wird man die Extraktion mit einem Trockensubstanzgehalt der Ausgangslösung zwischen 3 und 30 % durchführen, bevorzugt zwischen 10 und 20%, besonders bevorzugt bei 15%.

In einer speziellen Ausführungsform der vorliegenden Erfindung wird man den Extrakt nach erfolgter selektiver Anreicherung unter Erhalt eines Trockenextraktes trocknen. Die Trocknung erfolgt unter Verwendung von fachüblichen Verfahren, wie z.B. der Sprühbandtrocknung. Es ist jedoch ebenso möglich den angereicherten Extrakt ohne vorherige Trocknung weiterzuverwenden. Gegenstand der Erfindung ist auch ein Extrakt, der sich durch das erfindungsgemäße Verfahren herstellen lässt. Bevorzugt weist der Extrakt einen Mindestgehalt von 6 Gew.-% Quercetin-3-O-ß-Glucuronid auf. Typischerweise liegt der Gehalt an Gesamtanthocyanen bei maximal 1 Gew.-%, ebenfalls bezogen auf den Trockenextrakt.

Gegenstand der Erfindung ist auch eine Extraktzubereitung, die den erfindungsgemäßen Extrakt sowie mindestens einen physiologisch verträglichen Hilfsstoff enthält. Gegenstand der Erfindung ist auch eine pharmazeutische Zubereitung, Medizinprodukt oder Lebensmittel enthalten den erfindungsgemäßen Extrakt oder die Extraktzubereitung. Grundsätzlich kann die Applikationsform dieser Produkte intravasal, peroral, rektal oder transdermal sein, wobei diese topische oder systemische Wirkung haben kann.

Der Extrakt hat mindestens 15 Gew.-% an Flavonoiden und/oder mindestens 0,2 Gew.-% Anthocyanen. Eine verstärkte Inhibierung Permeabilitätstriggernder Zytokine kann im Vergleich zum Referenzextrakt (gemäß Stand der Technik) erreicht werden.

Die Erfindung wird durch die nachfolgenden nicht einschränkenden Beispiele näher erläutert.

### Verwendete Messmethoden

### Chemische Parameter

Die Gehaltsbestimmung der Flavonoide erfolgt mittels einer HPLC-Methode, welche die Flavonoide Isoquercitrin, Quercetin-3-O-ß-D-glucuronid und Kämpferol-3-O-ß-D-glucosid erfasst. Als stationäre Phase dient hierbei eine Superspher RP8 Trennsäule, mit 4 µm Partikeln, in der Dimension 125 x 4 mm. Die isokratische Trennung erfolgt bei einer Flussgeschwindigkeit von 1ml/min in einem Säulenofen bei 25°C. Der Eluent setzt sich aus 107 Teilen Tetrahydrofuran, 55 Teilen Acetonitril und 810 Teilen Phosphorsäure (0,003 mol/l) zusammen. Die Detektion erfolgt bei 360 nm.

Der Gehalt an Anthocyanen wird mit einer separaten HPLC-Methode analog Ph.Eur 6.4 Monographie 2394 "Fresh Bilberry fruit dry extract, refined and standardised" ermittelt.

### Pharmakologisches in-vitro-Modell zur Zytokin-Inhibierung

Humane Monozyten werden bei 37 °C und 5 % CO₂ für 24 h mit LPS (10ng/ml) stimuliert. Die Extrakte werden 30 Minuten vor Zugabe des LPS zugegeben, um zu prüfen, ob diese die LPS-Stimulation beeinflussen können. Es wurden Messkonzentrationen der Extrakte zwischen 10 - 300 µg/ml getestet. Nach 24 Stunden wurden die überstehenden Lösungen abgenommen, zentrifugiert und auf die Konzentrationen an PGE-2, IL-6, IL-1, und TNF-alpha mittels ELISAs / EIAs (Biotrend / Immunotools) gemäß Herstellervorgaben vermessen.

### Beispiel 1: wässriger Referenzextrakt (Stand der Technik)

2 kg Droge Folia Vitis vinifera Ph.Eur. werden mit 24 Litern Osmosewasser bei 75°C im Perkolator extrahiert. Das Eluat wird filtriert und im Unterdruck zu einem Dickextrakt mit ca. 50% Trockensubstanzanteil eingeengt. Der Spissum wird mit 4% Siliciumdioxid versetzt und bei 50°C im Vakuumtrockenschrank getrocknet. Der erhaltene Extrakt ist charakterisiert durch ein DEVnativ von 5:1, einen Flavonoidgehalt von 5,1 %, hiervon 3,5% Quercetin-3-O-beta-D-Glucuronid und einen Gehalt an Anthocyanen von 0,15%.

| [%] Inhibierung der Ausschüttung von | | | |
|---|---|---|---|
| TNF-alpha | IL-1β | IL-6 | PGE-2 |
| 50,4 ± 5,9 bei 50 µg/ml | 46,9 ± 1,9 bei 300 µg/ml | 42,5 ± 5,3 bei 50 µg/ml | 11,6 ± 5,5 bei 300 µg/ml |

### Beispiel 2: erfindungsgemäßer Adsorberextrakt (Basis wässrig)

2 kg Droge Folia Vitis vinifera Ph.Eur. werden mit 44 Litern Osmosewasser bei 75°C im Perkolator extrahiert. Das Eluat wird im Unterdruck zu einem Dickextrakt mit ca. 50% Trockensubstanz-Anteil eingeengt. Der Spissum wird über 16 Stunden kühl gelagert, und mit Osmosewasser auf einen Trockensubstanzanteil von 10% zurück verdünnt. Nach 24 Stunden werden die ausgefallenen Gerbstoffe (Tannine) abfiltriert und anschließend der Dünnextrakt auf Adsorbermaterial XAD7HP aufgebracht. Der beladene Adsorber wird mit Osmosewasser gewaschen und anschließend die Extraktkomponente mit Ethanol eluiert. Die Ethanolphase wird auf einen Trockensubstanzanteil von >50% eingedampft. Der erhaltene Spissum wird nativ bei 50°C im Vakuumtrockenschrank getrocknet. Der erhaltene Extrakt ist charakterisiert durch ein DEVnativ von 19:1, einen Flavonoidgehalt von 21,1%, hiervon 12,7% Quercetin-3-O-beta-D-Glucuronid und einen Gehalt an Anthocyanen von 0,7%.

| [%] Inhibierung der Ausschüttung von | | |
|---|---|---|
| Zytokin | IL-6 | PGE-2 |
| Extrakt gemäß Bsp. 2 | 42,5 ± 4,6 bei 10 µg/ml | 32,1 ± 7,1 bei 50 µg/ml |
| Extrakt gemäß Bsp. 1 | 42,5 ± 5,3 bei 50 µg/ml | 11,6 ± 5,5 bei 300 µg/ml |

Für den Adsorberextrakt nach Beispiel 2 konnte eine Steigerung der Zytokin-Inhibierung gegenüber dem Stand der Technik nach Bsp. 1 nachgewiesen werden.

Bei IL-6 ist eine um den Faktor 5 geringere Extraktkonzentration des erfindungsgemäßen Extraktes notwendig, um den gleichen anti-inflammatorischen Effekt zu erzielen.

Die Beeinflussung von PGE-2 ist noch stärker ausgeprägt. Bei einer Messkonzentration von 300µg/ml nach Bsp. 1 konnte nur eine 11,6%ige Inhibierung gemessen werden. Der erfindungsgemäße Extrakt nach Bsp. 2 erreichte mit einer 6-fach niedrigeren Konzentration von 50 µg/ml sogar eine 32,1%ige Inhibierung.

Diese beiden um den Faktor 5 bzw. Faktor 18 (3fach besserer %-Wert x Konzentrationsfaktor 6) liegen über dem Anreicherungsfaktor von 3,8 des erfindungsgemäßen Extraktes vs. Bsp. 1. Der Anreicherungsfaktor von 3,8 ergibt sich aus dem Quotienten des DEV nativ der zu vergleichenden Extrakte.

### Beispiel 3: erfindungsgemäßer Extrakt (mittels FI.-FI. Extraktion), Basis Wasser

2 kg Droge Folia Vitis vinifera Ph.Eur. werden mit 44 Litern Osmosewasser bei 75°C im Perkolator extrahiert. Das Eluat wird im Unterdruck zu einem Dickextrakt mit ca. 50% Trockensubstanz-Anteil eingeengt. Der Spissum wird über 16 Stunden kühl gelagert, und mit Osmosewasser auf einen Trockensubstanzanteil von 10% zurück verdünnt. Nach 24 Stunden wird die ausgefallene Tanninfraktion abfiltriert und anschließend der Dünnextrakt zweimal mit n-Butanol im Verhältnis 3:2 unterzogen. Die Butanolphase wird auf einen Trockensubstanzanteil von >50% eingedampft. Der erhaltene Spissum wird nativ bei 50°C im Vakuumtrockenschrank getrocknet. Der erhaltene Extrakt ist charakterisiert durch eine DEVnativ von 16:1, einen Flavonoidgehalt von 21,7%, hiervon 13,0% Quercetin-3-O-beta-D-Glucuronid und einen Gehalt an Anthocyanen von 0,43%. Die verbliebene Wasserphase charakterisiert sich über ein DEVnativ von 5:1 einen Flavonoidgehalt von < 0,1% und einen Gehalt an Anthocyanen unterhalb der Bestimmungsgrenze.

| | [%] Inhibierung der Ausschüttung von | | |
|---|---|---|---|
| | TNF-alpha | IL-1β | PGE 2 |
| BuOH-Phase Bsp. 3A | 52,2 ± 6,7 bei 10 µg/ml | 55,8 ± 6,6 bei 10 pq/ml | 24,3 ± 3,9 bei 50 µg/ml |
| H₂O-Phase Bsp.3B | 17,1 ± 4,8 bei 50 µg/ml | 39,8 ± 6,0 bei 300 µg/ml | 1,6 ± 4,6 bei 300 µg/ml |

Der Vergleich beider Phasen aus der Flüssig-Flüssig-Bearbeitung zeigt deutlich, dass die anti-inflammorischen Prinzipien in der organischen Phase (Butanol) angereichert werden können.

Für den butanolisch angereicherten Extrakt nach Beispiel 3A konnte eine Steigerung der Zytokin-Inhibierung von Faktor 15 (TNF-a), Faktor 42 (IL-1ß) und Faktor 91 (PGE-2) gegenüber der abgetrennten Wasserphase nach Bsp. 3B ermittelt werden

Demgegenüber steht nur ein Anreicherungsfaktor von 3,2 (Quotient des DEV nativ der zu vergleichenden Extrakte Bsp. 3A vs. Bsp. 3B).

Dies unterstreicht die selektive Anreicherung der aktiven Prinzipien aus der Wasserphase in die organische Phase.

Auch ist der erfindungsgemäße Extrakt nach Bsp. 3A dem Stand der Technik überlegen.

| [%] Inhibierung der Ausschüttung von | | |
|---|---|---|
| Zytokin | TNF-alpha | PGE 2 |
| Extrakt gemäß Bsp. 3A | 52,2 ± 6,7 bei 10 µg/ml | 24,3 ± 3,9 bei 50 µg/ml |
| Extrakt gemäß Bsp. 1 | 50,4 ± 5,9 bei 50 µg/ml | 11,6 ± 5,5 bei 300 µg/ml |

Für den Extrakt nach Beispiel 3A konnte eine Steigerung der Zytokin-Inhibierung gegenüber dem Stand der Technik nach Bsp. 1 nachgewiesen werden.

Bei TNF-alpha ist eine um den Faktor 5 geringere Extraktkonzentration des erfindungsgemäßen Extraktes 3A notwendig, um den gleichen anti-inflammatorischen Effekt zu erzielen.

Die Beeinflussung von PGE-2 ist noch stärker ausgeprägt. Bei einer Messkonzentration von 300µg/ml nach Bsp. 1 konnte nur eine 11,6%ige Inhibierung gemessen werden. Der erfindungsgemäße Extrakt nach Bsp. 2 erreichte mit einer 6-fach niedrigeren Konzentration von 50 µg/ml sogar eine 24,3%ige Inhibierung.

Diese beiden um den Faktor 5 bzw. Faktor 12 (2fach besserer %-Wert x Konzentrationsfaktor 6) liegen über dem Anreicherungsfaktor von 3,2 des erfindungsgemäßen Extraktes vs. Bsp. 1. Der Anreicherungsfaktor von 3,2 ergibt sich aus dem Quotienten des DEV nativ der zu vergleichenden Extrakte. Beispiel 4: Herstellung eines erfindungsgemässen Adsorberextraktes (Basis EtOH) 2 kg Droge Folia Vitis vinifera Ph.Eur. werden mit 24 Litern EtOH 20% V/V bei 75°C im Perkolator extrahiert. Das Eluat wird im Unterdruck zu einem Dickextrakt mit ca. 50% Trockensubstanzanteil eingeengt. Der Spissum wird über 16 Stunden kühl gelagert, und mit Osmosewasser auf einen Trockensubstanzanteil von 10% zurück verdünnt. Nach 24 Stunden werden die ausgefallenen Gerbstoffe abfiltriert und anschließend der Dünnextrakt auf Adsorbermaterial XAD7HP aufgebracht. Der beladene Adsorber wird mit Osmosewasser gewaschen und anschließend die Extraktkomponente mit Ethanol eluiert. Die Ethanolphase wird auf einen Trockensubstanzanteil von >50% eingedampft. Der erhaltene Spissum wird nativ bei 50°C im Vakuumtrockenschrank getrocknet. Der erhaltene Extrakt ist charakterisiert durch ein DEVnativ von 21:1 und einen Flavonoidgehalt von 25,1%, hiervon 17,0% Quercetin-3-O-beta-D-Glucuronid.

## Patentansprüche

1. Verfahren zur Herstellung eines Extraktes aus Blättern von Pflanzen der Ordnung *Vitales* umfassend folgende Schritte:
a) Bereitstellen einer Droge aus Blättern von Pflanzen der Ordnung *Vitales*
b) Extraktion der Droge mit einem Eluenten ausgewählt aus Wasser und Gemischen von Wasser mit Alkohol, um einen Rohextrakt zu erhalten,
c) Abtrennen des Rohextraktes von der extrahierten Droge,
d) zumindest teilweises Entfernen des Eluenten, um einen Dickextrakt zu erhalten,
e) Rücklösen des Dickextraktes in Wasser,
f) Abtrennen unlöslicher Bestandteile,
g) selektive Anreicherung sekundärer Pflanzeninhaltsstoffe durch
i. eine 2-Phasenextraktion als Festphasenextraktion mit einem Adsorberharz ausgewählt aus der Gruppe der nichtionischen hydrophoben Divinylbenzen-Copolymere, aliphatischen Esterpolymere und Formophenolpolymere mit einer durchschnittlichen inneren Porengröße von 30 bis 70 nm als feste Phase, wobei die Substanzen, die an den Adsorber binden eluiert werden und weiter verwendet werden
oder
ii. eine 2-Phasenextraktion als Flüssig-Flüssig-Extraktion wobei die zweite Phase eine mit Wasser nicht mischbare Flüssigkeit ist, ausgewählt aus der Gruppe der Alkohole, Ketone, Alkane oder Ester und die Fraktion, die in die zweite Phase übertritt weiter verwendet wird
so dass der Extrakt mindestens 15 Gew.-% an Flavonoiden und/oder mindestens 0,2 Gew.-% Anthocyane, bezogen auf den Trockenextrakt, enthält.

2. Verfahren nach Anspruch 1, **dadurch gekennzeichnet, dass** die Droge getrocknet und/oder zerkleinert wird.

3. Verfahren nach Anspruch 1 oder 2, **dadurch gekennzeichnet, dass** der Extrakt getrocknet wird.

4. Verfahren nach einem der Ansprüche 1 bis 3, wobei der Eluent Wasser oder eine Ethanol-Wassermischung ist, die 5 - 95 % (V/V) Ethanol enthält.

5. Verfahren nach Anspruch 4, wobei der Eluent 25 - 50 % (V/V) Ethanol enthält.

6. Verfahren nach einem der vorhergehenden Ansprüche, wobei der Eluent zur Extraktion auf eine Temperatur von 40 - 80°C erwärmt wird, bevorzugt 15 - 25°C unterhalb der Siedetemperatur.

7. Extrakt hergestellt nach einem der Ansprüche 1 bis 6.

8. Extraktzubereitung, enthaltend einen Extrakt nach Anspruch 7 sowie mindestens einen physiologisch verträglichen Hilfsstoff.

9. Pharmazeutische Zubereitung, Medizinprodukt oder Lebensmittel enthaltend einen Extrakt nach Anspruch 7 oder eine Extraktzubereitung nach Anspruch 8.

10. Pharmazeutische Zubereitung, Medizinprodukt oder Lebensmittel nach Anspruch 9, wobei die Applikationsform intravasal, peroral, rektal oder transdermal ist.

11. Pharmazeutische Zubereitung, Medizinprodukt oder Lebensmittel nach Anspruch 10, wobei die intravasale, rektale oder transdermale Applikation topische oder systemische Wirkung hat.

## Claims

1. A process for preparing an extract from leaves of plants of the order Vitales, comprising the following steps:
a) providing a crude drug from leaves of plants of the order Vitales;
b) extracting the crude drug with an eluent selected from water and mixtures of water with alcohol to obtain a raw extract;
c) separating the raw extract from the extracted crude drug;
d) at least partially removing the eluent to obtain a thick extract;
e) redissolving the thick extract in water;
f) separating off insoluble components;
g) selectively enriching secondary plant substances by
i.) a two-phase extraction as a solid-phase extraction with an adsorber resin selected from the group of non-ionic hydrophobic divinylbenzene copolymers, aliphatic ester polymers and formophenol polymers with an average inner pore size of from 30 to 70 nm as the solid phase, wherein the substances binding to the adsorber are eluted and used further; or
ii.) a two-phase extraction as a liquid-liquid extraction, wherein the second phase is a liquid immiscible with water, selected from the group of alcohols, ketones, alkanes or esters, and the fraction transferred into the second phase is used further;
so that the extract contains at least 15% by weight flavonoids and/or at least 0.2% by weight anthocyans, based on the dry extract.

2. The process according to claim 1, **characterized in that** said crude drug is dried and/or comminuted.

3. The process according to claim 1 or 2, **characterized in that** said extract is dried.

4. The process according to any of claims 1 to 3, wherein the eluent is water or an ethanol-water mixture containing from 5 to 95% (v/v) ethanol.

5. The process according to claim 4, wherein the eluent contains from 25 to 50% (v/v) ethanol.

6. The process according to any of the preceding claims, wherein the eluent is heated to a temperature of 40-80 °C for extraction, preferably to 15-25 °C below the boiling temperature.

7. An extract prepared according to any of claims 1 to 6.

8. An extract formulation containing an extract according to claim 7 and at least one physiologically tolerable auxiliary agent.

9. A pharmaceutical formulation, medicinal product or food containing an extract according to claim 7 or an extract formulation according to claim 8.

10. The pharmaceutical formulation, medicinal product or food according to claim 9, wherein the dosage form is an intravasal, peroral, rectal or transdermal form.

11. The pharmaceutical formulation, medicinal product or food according to claim 10, wherein said intravasal, rectal or transdermal administration has a topical or systemic effect.

## Revendications

1. Procédé de production d'un extrait à partir de feuilles de plantes de l'ordre Vitales, comprenant les étapes suivantes :
a) préparation d'une drogue à partir de feuilles de plantes de l'ordre *Vitales,*
b) extraction de la drogue avec un éluant sélectionné parmi l'eau et des mélanges d'eau avec de l'alcool pour obtenir un extrait brut,
c) séparation opérée entre l'extrait brut et la drogue extraite,
d) enlèvement au moins partiel de l'éluant pour obtenir un extrait épais,
e) redissolution de l'extrait épais dans de l'eau,
f) séparation des composants non solubles,
g) enrichissement sélectif de composantes végétales secondaires par
i. une extraction biphasée en tant qu'extraction en phase solide avec une résine d'absorption sélectionnée parmi le groupe des copolymères de divinylbenzène hydrophobes non ioniques, des polymères d'ester aliphatiques et des polymères formophénoliques avec une dimension de pores intérieure moyenne de 30 à 70 nm en tant que phase solide, les substances qui se lient à l'adsorbeur étant éluées et utilisées ultérieurement
ou
ii. une extraction biphasée en tant qu'extraction liquide-liquide, la deuxième phase étant un liquide non miscible avec l'eau, sélectionné parmi le groupe des alcools, cétones, alcanes ou esters, et la fraction qui passe dans la deuxième phase étant utilisée ultérieurement
de telle sorte que l'extrait contient au moins 15 %-poids de flavonoïdes et/ou au moins 0,2 %-poids d'anthocyanes, rapportés à l'extrait sec.

2. Procédé selon la revendication 1, **caractérisé en ce que** la drogue est séchée et/ou broyée.

3. Procédé selon la revendication 1 ou 2, **caractérisé en ce que** l'extrait est séché.

4. Procédé selon l'une des revendications 1 à 3, **caractérisé en ce que** l'éluant est de l'eau ou un mélange eau-éthanol, qui contient 5 à 95 % (V/V) d'éthanol.

5. Procédé selon la revendication 4, l'éluant contenant 25 à 50 % (V/V) d'éthanol.

6. Procédé selon l'une des revendications précédentes, **caractérisé en ce que** l'éluant est réchauffé pour l'extraction à une température de 40 à 80 °C, de préférence de 15 à 25 °C au-dessous de la température d'ébullition.

7. Extrait produit selon l'une des revendications 1 à 6.

8. Préparation d'extrait, contenant un extrait selon la revendication 7 ainsi qu'au moins un adjuvant physiologiquement compatible.

9. Préparation pharmaceutique, produit médicinal ou aliment contenant un extrait selon la revendication 7 ou une préparation d'extrait selon la revendication 8.

10. Préparation pharmaceutique, produit médicinal ou aliment selon la revendication 9, la forme d'application étant intravasculaire, orale, rectale ou transdermique.

11. Préparation pharmaceutique, produit médicinal ou aliment selon la revendication 10, l'application intravasculaire, rectale ou transdermique ayant un effet topique ou systémique.
